(19) Europäisches Patentamt
European Patent Office
Office européen des brevets

(11) **EP 4 302 997 A1**

(12) **EUROPEAN PATENT APPLICATION**
published in accordance with Art. 153(4) EPC

(43) Date of publication:
**10.01.2024 Bulletin 2024/02**

(21) Application number: **22763263.5**

(22) Date of filing: **01.03.2022**

(51) International Patent Classification (IPC):
***B32B 27/32*** *(2006.01)*

(52) Cooperative Patent Classification (CPC):
**B32B 27/32**

(86) International application number:
**PCT/JP2022/008633**

(87) International publication number:
**WO 2022/186208 (09.09.2022 Gazette 2022/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.03.2021 JP 2021032693
09.11.2021 JP 2021182811**

(71) Applicant: **Mitsui Chemicals, Inc.
Tokyo 104-0028 (JP)**

(72) Inventor: **KATAYAMA, Yoshiyuki
Tokyo 104-0028 (JP)**

(74) Representative: **J A Kemp LLP
80 Turnmill Street
London EC1M 5QU (GB)**

(54) **MULTILAYER FILM, CONTAINER, PACK FOR CELL CULTURE AND METHOD FOR PRODUCING MULTILAYER FILM**

(57)     A multilayer film includes a layer that includes a 4-methyl-1-pentene-based polymer, and a layer that includes a polyolefin. The layer that includes a 4-methyl-1-pentene-based polymer and the layer that includes a polyolefin contact each other at at least a portion thereof, the inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

EP 4 302 997 A1

**Description**

Technical Field

The present disclosure relates to a multilayer film, a container, a pack for cell culture, and a method for producing a multicellular film.

Background Art

4-methyl-1-pentene polymers have bulky functional groups, and thus have a lower density than that of other thermoplastic polyolefin multilayer films. Therefore, multilayer films containing a 4-methyl-1-pentene polymer have high gas permeability with respect to oxygen gas, carbon dioxide gas, and the like, and are being developed as gas permeable multilayer films such as packaging materials for fresh foods or the like (see, for example, Patent Document 1).

It is also known that 4-methyl-1-pentene polymers have a high melting point and a mold release property. Therefore, multilayer films mainly composed of a 4-methyl-1-pentene polymer (hereinafter sometimes referred to as "4-methyl-1-pentene-based polymer multilayer film") require a high heat-sealing temperature and provide a low heat-seal strength. In consideration of this, a method in which a multilayer film composed of another thermoplastic resin such as an olefin-based polymer (hereinafter sometimes referred to as an "olefin-based polymer multilayer film") is superposed on a 4-methyl-1-pentene-based polymer multilayer film, has been studied as a method for imparting heat-sealability to the 4-methyl-1-pentene-based polymer multilayer film (see, for example, Patent Document 2).

Further, multilayer films having gas permeability imparted by making holes in, for example, a polypropylene multilayer film with a laser or a heated needle are commercially available (see, for example, Patent Document 3).

Patent Document 1: Japanese Patent Application Laid-open (JP-A) No. H11-301691
Patent Document 2: Japanese Patent Application Laid-open (JP-A) No. 2000-189051
Patent Document 3: Japanese Patent Application Laid-open (JP-A) No. 2015-224037

SUMMARY OF INVENTION

Problem to be solved by invention

As described above, 4-methyl-1-pentene polymers have a high gas permeability. However, 4-methyl-1-pentene resins are non-polar and have low wettability, as a result of which 4-methyl-1-pentene resins have mold release properties. Therefore, there are cases in which 4-methyl-1-pentene resins are difficult to adhere.

For example, in the case of producing a multilayer film using a 4-methyl-1-pentene resin and producing a packaging material from the multilayer film, the packaging material is required to have heat-sealability in some cases. However, since 4-methyl-1-pentene resins have mold release properties, it is difficult to obtain a sufficient heat-seal strength.

In addition, a multilayer film or laminated multilayer film that is mainly composed of a 4-methyl-1-pentene polymer has a high heat-sealing temperature. Therefore, when such a multilayer film or laminated multilayer film is used as, for example, a multilayer film for fresh foods, there is a possibility that the taste of the content may deteriorate or liquid leakage or breakage may occur at heat-sealed portions. Accordingly, such a multilayer film or laminated multilayer film has a drawback of being unsuitable for practical use.

A problem to be solved by one embodiment of the present disclosure is to provide a multilayer film, a container, or a pack for cell culture, which has excellent gas permeability and excellent heat-sealability at low temperatures, and a method of producing a multilayer film.

Means for Solving the Problem

Means for solving the above problem include the following aspects.

<1> A multilayer film, comprising a layer that includes a 4-methyl-1-pentene-based polymer, and a layer that includes a polyolefin, wherein the layer that includes a 4-methyl-1-pentene-based polymer and the layer that includes a polyolefin contact each other at at least a portion thereof, the inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

<2> The multilayer film according to <1>, wherein the layer that includes a 4-methyl-1-pentene-based polymer has a content of the 4-methyl-1-pentene-based polymer of from 70 parts by mass to 100 parts by mass.

<3> The multilayer film according to <1> or <2>, wherein the polyolefin includes at least one selected from the group consisting of an unsaturated carboxylic acid modified ethylene-$\alpha$-olefin copolymer, an ethylene-unsaturated carboxylic acid-based copolymer, an ethylene-vinyl acetate copolymer, a silane modified ethylene-vinyl acetate copolymer, an ionomer of an unsaturated carboxylic acid modified ethylene-$\alpha$-olefin copolymer, and an ionomer of an ethylene-unsaturated carboxylic acid-based copolymer.

<4> The multilayer film according to any one of <1> to <3>, wherein the layer that includes a 4-methyl-1-pentene-based polymer has a thickness of from 5 $\mu$m to 50 $\mu$m.

<5> The multilayer film according to any one of <1> to <4>, wherein the layer that includes a polyolefin has a thickness of from 1 $\mu$m to 15 $\mu$m.

<6> The multilayer film according to any one of <1> to <5>, wherein at least one side of the layer that includes a 4-methyl-1-pentene-based polymer has been subjected to at least one treatment selected from the group consisting of plasma treatment, corona treatment, ozone treatment, UV treatment, and flame treatment.

<7> The multilayer film according to any one of <1> to <6>, wherein the multilayer film is obtained by extrusion-laminating the layer that includes a polyolefin onto the layer that includes a 4-methyl-1-pentene-based polymer.

<8> The multilayer film according to any one of <1> to <7>, wherein the multilayer film is an oxygen permeable multilayer film or a carbon dioxide permeable multilayer film.

<9> The multilayer film according to any one of <1> to <8>, wherein the multilayer film has an oxygen permeability of from 11,000 mL/m$^2$·day·atm to 100,000 mL/m$^2$·day·atm, and a carbon dioxide permeability of from 25,000 mL/m$^2$·day·atm to 250,000 mL/m$^2$·day·atm.

<10> The multilayer film according to any one of <1> to <9>, wherein the multilayer film has a hydrogen permeability of from 10,000 mL/m$^2$·day·atm to 300,000 mL/m$^2$·day·atm.

<11> The multilayer film according to any one of <1> to <10>, wherein the multilayer film has a moisture permeability of from 10 g/m$^2$·day to 80 g/m$^2$·day.

<12> The multilayer film according to any one of <1> to <11>, wherein irregularities are formed on a surface of the layer that includes a polyolefin.

<13> A container, comprising the multilayer film according to any one of <1> to <12>, wherein the container is configured to be sealed by heat-sealing the layers that include a polyolefin included in the multilayer film(s).

<14> A pack for cell culture, comprising the multilayer film according to any one of <1> to <13>.

<15> A method of producing a multilayer film, the method comprising:

a treatment step of performing a surface treatment on at least one side of a base material that includes a 4-methyl-1-pentene-based polymer; and

a lamination step of laminating a polyolefin onto the side of the base material that has been subjected to the surface treatment, to produce a multilayer film,

wherein, in the multilayer film, the inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

<16> The method of producing a multilayer film according to <15>, wherein the surface treatment includes at least one treatment selected from the group consisting of plasma treatment, corona treatment, ozone treatment, UV treatment, and flame treatment

<17> The method of producing a multilayer film according to <15> or <16>, wherein the lamination step includes subjecting the polyolefin to ozone treatment, and laminating the polyolefin onto the side of the base material that has been subjected to the surface treatment while a side of the polyolefin that has been subjected to the ozone treatment contacts the side of the base material that has been subjected to the surface treatment.

<18> The method of producing a multilayer film according to any one of <15> to <17>, wherein the lamination step is a step of laminating the polyolefin onto the base material that includes a 4-methyl-1-pentene-based polymer, using extrusion lamination.

Advantageous Effect of Invention

According to an embodiment of the present disclosure, a multilayer film, a container, or a pack for cell culture, which has excellent gas permeability and excellent heat-sealability at low temperatures, and a method of producing a multilayer film are provided.

MODES FOR CARRYING OUT INVENTION

Specific embodiments according to the present disclosure are described below in detail. However, the present disclosure is by no means limited to the embodiments described below, and modifications may be made, as appropriate, within the purpose of the present disclosure.

In the present specification, any numerical range indicated using "to" denotes a range including numerical values noted before and after "to" as the lower limit value and the upper limit value.

In the present specification, a "side" of a member means a "principal face" of the member, unless specified otherwise.

In the present specification, the term "adhesion" denotes a concept that encompasses pressure-sensitive adhesion.

In the present specification, the term "multilayer film" denotes a concept that encompasses not only those generally referred to as "multilayer films" but also those generally referred to as "sheets".

In a case in which plural substances corresponding to a component of interest are present in a composition, the amount of the component in the composition described in the present specification means the total amount of the plural substances present in the composition, unless otherwise specified.

«Multilayer Film»

The multilayer film according to the present disclosure includes a layer that includes a 4-methyl-1-pentene-based polymer, and a layer that includes a polyolefin, wherein the layer that includes a 4-methyl-1-pentene-based polymer and the layer that includes a polyolefin contact each other at at least a portion thereof, the inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

The multilayer film according to the present disclosure has excellent gas permeability and excellent heat-sealability at low temperatures, owing to the above-described configuration adopted in the multilayer film.

It has hitherto been highly difficult to heat-seal a 4-methyl-1-pentene-based polymer since 4-methyl-1-pentene-based polymers have high mold release properties and high heat resistance. For example, when heat-sealing of a 4-methyl-1-pentene-based polymer is attempted, it has been difficult to obtain sufficient heat-sealability even upon application of a high temperature of about 250 °C. Therefore, it has been difficult to obtain sufficient heat-sealability at a low temperature of, for example, about 120 °C. Conceivable measures may include producing a multilayer film by means of laminating a 4-methyl-1-pentene-based polymer and a heat-seal layer with another layer, such as an adhesion layer, disposed therebetween. However, since 4-methyl-1-pentene-based polymers have high mold release properties and high water repellency, laminating a 4-methyl-1-pentene-based polymer and the other layer, such as an adhesion layer, together is also difficult.

Further, although 4-methyl-1-pentene-based polymers have excellent gas permeability, the excellent gas permeability tends to deteriorate when the thickness of a multilayer film including a 4-methyl-1-pentene-based polymer increases.

Thus, it has been difficult to obtain a multilayer film that includes a layer including a 4-methyl-1-pentene-based polymer, and that achieves both of gas permeability and heat-sealability at low temperatures.

In the multilayer film according to the present disclosure, a layer that includes a 4-methyl-1-pentene-based polymer and a layer that includes a polyolefin contact each other at at least a portion thereof. In the portion at which the layer that includes a 4-methyl-1-pentene-based polymer and the layer that includes a polyolefin contact each other, the layer that includes a 4-methyl-1-pentene-based polymer and the layer that includes a polyolefin are directly adhered to each other. In other words, in the multilayer film according to the present disclosure, the layer that includes a 4-methyl-1-pentene-based polymer and the layer that includes a polyolefin directly contact each other at at least a portion thereof.

In the multilayer film according to the present disclosure, the area of the portion at which the layer that includes a 4-methyl-1-pentene-based polymer and the layer that includes a polyolefin contact each other is preferably 50% or more, more preferably 70% or more, and still more preferably 90% or more, with respect to the total area of the multilayer film.

Further, in the multilayer film according to the present disclosure, it is also preferable that the layer that includes a 4-methyl-1-pentene-based polymer and the layer that includes a polyolefin contact each other at the entire face.

A method that can be used for direct adhesion is, for example, the direct lamination method described below.

Adhering a layer that includes a 4-methyl-1-pentene-based polymer and a layer that includes a polyolefin has been performed generally by disposing, between the layers, a layer that exhibits adhesiveness. The reason is that 4-methyl-1-pentene-based polymers exhibit low adhesion power, and, therefore, it has been difficult to directly adhere the layer that includes a 4-methyl-1-pentene-based polymer and the layer that includes a polyolefin.

However, in the multilayer film according to the present disclosure, although a layer that exhibits adhesiveness may be present between the layer that includes a 4-methyl-1-pentene-based polymer and the layer that includes a polyolefin, the presence of the layer that exhibits adhesiveness between the layer that includes a 4-methyl-1-pentene-based polymer and the layer that includes a polyolefin is not essential.

The thickness of the multilayer film is preferably, for example, from 10 $\mu$m to 100 $\mu$m. The thickness of the multilayer film is preferably, for example, 80 $\mu$m or less, and more preferably 70 $\mu$m or less, from the viewpoint of gas permeability.

The thickness of the multilayer film is preferably, for example, 15 $\mu$m or more, and more preferably 20 $\mu$m or more, from the viewpoint of maintaining the balance between gas permeability and heat-sealability.

<Layer That Includes 4-methyl-1-pentene-based Polymer>

The multilayer film according to the present disclosure includes a layer that includes a 4-methyl-1-pentene-based polymer (also referred to as a "4-methyl-1-pentene layer"). The 4-methyl-1-pentene-based polymer may be a homopolymer of 4-methyl-1-pentene, or may be a copolymer of 4-methyl-1-pentene and another monomer. The copolymer of 4-methyl-1-pentene and another monomer is preferably the 4-methyl-1-pentene-based polymer (A1) described below.

(4-methyl-1-pentene-based Polymer (A1))

A 4-methyl-1-pentene-based polymer (A1) refers to a copolymer that includes a structural unit derived from 4-methyl-1-pentene and a structural unit derived from ethylene or an $\alpha$-olefin having 3 to 20 carbon atoms (excluding 4-methyl-1-pentene).

Assuming that the total of structural units derived from 4-methyl-1-pentene and structural units derived from ethylene or an $\alpha$-olefin having 3 to 20 carbon atoms is 100 mol%, the structural units derived from 4-methyl-1-pentene may be in the range of from 60 mol% to 99 mol%, preferably in the range of from 65 mol% to 98 mol%, and more preferably in the range of from 65 mol% to 97 mol%.

When the structural units derived from 4-methyl-1-pentene are in an amount of 60 mol% or more, a multilayer film having a higher gas permeability can be obtained. When the structural units derived from 4-methyl-1-pentene are in an amount of 99 mol% or less, a multilayer film having more favorable mechanical properties, such as multilayer film elongation, can be obtained.

Examples of the ethylene or $\alpha$-olefin having 3 to 20 carbon atoms include linear or branched $\alpha$-olefins, cyclic olefins, aromatic vinyl compounds, conjugate dienes, non-conjugate polyenes, and functionalized vinyl compounds.

Examples of the linear or branched $\alpha$-olefins include: linear $\alpha$-olefins such as ethylene, propylene, 1-butene, 1-pentene, 1-hexene, 1-octene, 1-decene, 1-dodecene, 1-tetradecene, 1-hexadecene, 1-octadecene, and 1-eicosene; and branched $\alpha$-olefins such as 3-methyl-1-butene, 3-methyl-1-pentene, 3-ethyl-1-pentene, 4,4-dimethyl-1-pentene, 4-methyl-1-hexene, 4,4-dimethyl-1-hexene, 4-ethyl-1-hexene, and 3-ethyl-1-hexene. The linear $\alpha$-olefins may have from 2 to 20 carbon atoms, and preferably have from 2 to 10 carbon atoms. The branched $\alpha$-olefins preferably have from 5 to 20 carbon atoms, and more preferably have from 5 to 10 carbon atoms.

Examples of the cyclic olefins include cyclopentene, cycloheptene, norbornene, 5-methyl-2-norbornene, tetracyclododecene, vinyl norbornene, vinyl cyclohexane, and other cyclic olefins. The cyclic olefins may have from 4 to 20 carbon atoms, and preferably have from 5 to 15 carbon atoms.

Examples of the aromatic vinyl compounds include: styrene; and mono- or polyalkylstyrenes such as $\alpha$-methylstyrene, o-methylstyrene, m-methylstyrene, p-methylstyrene, o,p-dimethylstyrene, o-ethylstyrene, m-ethylstyrene, and p-ethylstyrene.

Examples of the conjugate dienes include 1,3-butadiene, isoprene, chloroprene, 1,3-pentadiene, 2,3-dimethylbutadiene, 4-methyl-1,3-pentadiene, 1,3-hexadiene, 1,3-octadiene, and other conjugate dienes. The conjugate dienes may have from 4 to 20 carbon atoms, and preferably have from 4 to 10 carbon atoms.

Examples of the non-conjugate polyenes include 1,4-pentadiene, 1,4-hexadiene, 1,5-hexadiene, 1,4-octadiene, 1,5-octadiene, 1,6-octadiene, 1,7-octadiene, 2-methyl-1,5-hexadiene, 6-methyl-1,5-heptadiene, 7-methyl-1,6-octadiene, 4-ethylidene-8-methyl-1,7-nonadiene, 4,8-dimethyl-1,4,8-decatriene (DMDT), dicyclopentadiene, cyclohexadiene, dicyclooctadiene, methylene norbornene, 5-vinylnorbornene, 5-ethylidene-2-norbomene, 5-methylene-2-norbornene, 5-isopropylidene-2-norbornene, 6-chloromethyl-5-isopropenyl-2-norbornene, 2,3-diisopropylidene-5-norbornene, 2-ethylidene-3-isopropylidene-5-norbornene, 2-propenyl-2,2-norbornadiene, and other non-conjugate polyenes. The non-conjugate polyenes may have from 5 to 20 carbon atoms, and preferably have from 5 to 10 carbon atoms.

Examples of the functionalized vinyl compounds include: hydroxy group-containing olefins; halogenated olefins; unsaturated carboxylic acids such as acrylic acid, propionic acid, 3-butenoic acid, 4-pentenoic acid, 5-hexenoic acid, 6-heptenoic acid, 7-octenoic acid, 8-nonenoic acid, and 9-decenoic acid; unsaturated amines such as allylamine, 5-hexeneamine, and 6-hepteneamine; unsaturated acid anhydrides such as (2,7-octadienyl)succinic anhydride, pentapropenyl succinic anhydride, and acid anhydrides of the above-described unsaturated carboxylic acids; halogenated products of the above-described unsaturated carboxylic acids; and unsaturated epoxy compounds such as 4-epoxy-1-butene, 5-epoxy-1-pentene, 6-epoxy-1-hexene, 7-epoxy-1-heptene, 8-epoxy-1-octene, 9-epoxy-1-nonene, 10-epoxy-1-decene, and 11-epoxy-1-undecene.

The hydroxyl group-containing olefin may be any olefin that has a hydroxyl group, without particular restrictions. The hydroxyl group-containing olefin is preferably a terminally hydroxylated olefin compound.

Examples of the terminally hydroxylated olefin compound include: hydroxylated α-olefins such as vinyl alcohol, allyl alcohol, hydroxylated 1-butene, hydroxylated 1-pentene, hydroxylated 1-hexene, hydroxylated 1-octene, hydroxylated 1-decene, hydroxylated 1-dodecene, hydroxylated 1-tetradecene, hydroxylated 1-hexadecene, hydroxylated 1-octadecene, and hydroxylated 1-eicosene; branched hydroxylated α-olefins such as hydroxylated 3-methyl-1-butene, hydroxylated 4-methyl-1-pentene, hydroxylated 3-methyl-1-pentene, hydroxylated 3-ethyl-1-pentene, hydroxylated 4,4-dimethyl-1-pentene, hydroxylated 4-methyl-1-hexene, hydroxylated 4,4-dimethyl-1-hexene, hydroxylated 4-ethyl-1-hexene, and hydroxylated 3-ethyl-1-hexene.

The hydroxylated α-olefins may have from 4 to 20 carbon atoms, and preferably have from 2 to 10 carbon atoms. The branched hydroxylated hydroxylated α-olefins preferably have from 5 to 20 carbon atoms, and more preferably have from 5 to 10 carbon atoms.

Examples of the halogenated olefins include: linear halogenated α-olefins such as halogenated 1-butene, halogenated 1-pentene, halogenated 1-hexene, halogenated 1-octene, halogenated 1-decene, halogenated 1-dodecene, halogenated 1-tetradecene, halogenated 1-hexadecene, halogenated 1-octadecene, and halogenated 1-eicosene; and branched halogenated α-olefins such as halogenated 3-methyl-1-butene, halogenated 4-methyl-1-pentene, halogenated 3-methyl-1-pentene, halogenated 3-ethyl-1-pentene, halogenated 4,4-dimethyl-1-pentene, halogenated 4-methyl-1-hexene, halogenated 4,4-dimethyl-1-hexene, halogenated 4-ethyl-1-hexene, and halogenated 3-ethyl-1-hexene.

The linear halogenated α-olefins may have from 4 to 20 carbon atoms, and preferably have from 4 to 10 carbon atoms. The branched halogenated α-olefins may have from 5 to 20 carbon atoms, and more preferably have from 5 to 10 carbon atoms.

The 4-methyl-1-pentene-based polymer (A1) may include only one kind of structural unit derived from ethylene or an α-olefin having from 3 to 20 carbon atoms, or may include two or more kinds of structural units selected from the group consisting of ethylene and α-olefins having from 3 to 20 carbon atoms.

As the α-olefins having from 2 to 20 carbon atoms other than 4-methyl-1-pentene, at least one selected from the group consisting of ethylene, propylene, 1-butene, 1-pentene, 1-hexene, 1-heptene, 1-octene, 1-nonene, 1-decene, 1-hexadecene, and 1-octadecene is preferable, at least one selected from the group consisting of propylene, 1-butene, 1-hexene, and 1-octene is more preferable, and at least one selected from the group consisting of ethylene, propylene, and 1-butene is still more preferable, and ethylene and propylene are particularly preferable, from the viewpoint of controlling the polymerizability, and properties, particularly the melting point Tm, of the resultant polymer.

The content ratio (mol%) of structural units included in the 4-methyl-1-pentene-based polymer (A1) can be measured according to the following method.

-Conditions-

Measurement instrument: nuclear magnetic resonance instrument (type ECP500, manufactured by JEOL Ltd.)
Nuclide to be observed: 13C (125 MHz)
Sequence: single pulse proton decoupling
Pulse width: 4.7 μsec. (45° pulses)
Repetition Time: 5.5 sec.
Accumulated scans: 10,000 times or more
Solvent: orthodichlorobenzene / deuterated benzene (volume ratio: 80/20) mixed solvent Sample concentration: 55 mg / 0.6 mL
Measurement temperature: 120 °C
Chemical shift standard: 27.50 ppm

It is preferable that the 4-methyl-1-pentene-based polymer (A1) has a melting point Tm as measured by a differential scanning calorimeter of 199 °C or lower, or is a polymer of which melting point Tm is not observed when measured by a differential scanning calorimeter.

The expression, "melting point Tm is not observed", means that a crystal melting peak having a crystal melting heat of 1 J/g or more is not substantially observed in the range of from -150°C to 200°C.

In order to adjust the melting point Tm of the 4-methyl-1-pentene-based polymer (A1) as measured by a differential scanning calorimeter to 199 °C or lower, or provide a 4-methyl-1-pentene-based polymer (A1) of which melting temperature Tm is not observed by a differential scanning calorimeter, it is preferable to adjust the amount of structural units derived from 4-methyl-1-pentene to a range of from 60 mol% to 99 mol%, assuming that the total amount of structural units derived from 4-methyl-1-pentene and structural units derived from ethylene or an $\alpha$-olefin having 3 to 20 carbon atoms (excluding 4-methyl-1-pentene) included in the 4-methyl-1-pentene-based polymer (A1) is 100 mol%.

A multilayer film that is capable of shaping at low temperatures and heat sealing at low temperatures can be obtained by satisfying the condition that the melting point Tm of the 4-methyl-1-pentene-based polymer (A1) is 199 °C or lower or the condition that the melting point Tm of the 4-methyl-1-pentene-based polymer (A1) is not observed.

It is preferable that the melting point Tm of the 4-methyl-1-pentene-based polymer (A1) is from 100 °C to 180 °C, or that the melting point Tm of the 4-methyl-1-pentene-based polymer (A1) is not observed.

The melting point Tm of the 4-methyl-1-pentene-based polymer (A1) is a value obtained by a measurement using a differential scanning calorimetry (DSC) according to the following method.

About 5 mg of the 4-methyl-1-pentene-based polymer (A1) is placed in a measurement aluminum pan of a differential scanning calorimeter (DSC model 220 C) manufactured by Seiko Instrument Inc., which is then hermetically sealed, and the 4-methyl-1-pentene-based polymer (A1) is heated from room temperature to 200 °C at a rate of 10 °C/min. In order to completely melt the 4-methyl-1-pentene-based polymer (A1), the 4-methyl-1-pentene-based polymer (A1) is held at 200 °C for 5 minutes, and then cooled to - 50 °C at a rate of 10 °C/min. The 4-methyl-1-pentene-based polymer (A1) is left at -50 °C for 5 minutes, and then second heating is carried out to 200 °C at a rate of 10 °C/min. The peak temperature (°C) during the second heating is taken as the melting point Tm of the 4-methyl-1-pentene-based polymer (A1). If plural peaks are detected, the peak detected at the highest temperature among them will be adopted.

The 4-methyl-1-pentene-based polymer (A1) preferably satisfies, in addition to the foregoing requirements, at least one of (i) the conditions for intrinsic viscosity [$\eta$], (ii) the conditions for weight average molecular weight (Mw), (iii) the conditions for melt flow rate (MFR), or (iv) the conditions for density, which are described below.

-Intrinsic Viscosity [$\eta$]-

The intrinsic viscosity [$\eta$] of the 4-methyl-1-pentene-based polymer (A1) as measured at 135 °C in a decalin solvent is preferably from 0.5 dL/g to 5.0 dL/g, and more preferably from 0.5 dL/g to 4.0 dL/g.

When the intrinsic viscosity [$\eta$] of the 4-methyl-1-pentene-based polymer (A1) is within the above-described range, the multilayer film is less sticky due to the amount of low-molecular-weight molecules being small, and extrusion multilayer film molding can be carried out.

The intrinsic viscosity [$\eta$] is a value as measured according to the following method, using an ubbelohde viscometer. About 20 mg of the 4-methyl-1-pentene-based polymer (A1) is dissolved in 25 ml of decalin, and then specific viscosity $\eta$sp is measured in an oil bath at 135 °C, using an ubbelohde viscometer. After the decalin solution is diluted by addition of 5 ml of decalin, specific viscosity $\eta$sp is measured in the same manner as that described above. This dilution operation is further repeated twice, and the value of $\eta$sp/C obtained by extrapolation to a concentration (C) of 0 is determined as the intrinsic viscosity [$\eta$] (unit: dL/g) (see the following Expression 1).

$$[\eta] = \lim(\eta sp/C)(C \to 0) \qquad \text{Expression 1}$$

-Weight Average Molecular Weight (Mw)-

The weight average molecular weight (Mw) of the 4-methyl-1-pentene-based polymer (A1) is preferably from $1 \times 10^4$ to $2 \times 10^6$, and more preferably from $1 \times 10^4$ to $1 \times 10^6$, from the viewpoint of formability of the multilayer film.

The 4-methyl-1-pentene-based polymer (A1) preferably has a molecular weight distribution (Mw/Mn) of from 1.0 to 3.5, more preferably from 1.1 to 3.0, from the viewpoint of stickiness and outer appearance of the multilayer film.

The weight average molecular weight (Mw) of the 4-methyl-1-pentene-based polymer (A1) and the molecular weight distribution (Mw/Mn) of the 4-methyl-1-pentene-based polymer (A1) expressed as a ratio of weight average molecular weight (Mw) to number average molecular weight (Mn) are values as determined according to the standard polystyrene conversion method using the following gel permeation chromatography (GPC).

•• Conditions ••

Measurement apparatus: GPC (model ALC/GPC150-Cplus, having an integrated differential refractometer detector; manufactured by Waters)
Columns: two GMH6-HT columns (manufactured by Tosoh Corporation) and two GMH6-HTL columns (manufactured by Tosoh Corporation), which are connected in series
Eluent: o-dichlorobenzene
Column temperature: 140 °C
Flow rate: 1.0 ml/min.

-Melt Flow Rate (MFR)-

The melt flow rate (MFR) of the 4-methyl-1-pentene-based polymer (A1) is preferably from 0.1 g/10 min. to 100 g/10 min., more preferably from 0.5 g/10 min. to 50 g/10 min., and still more preferably 0.5 g/10 min. to 30 g/10 min., from the viewpoint of flowability at the time of shaping of the multilayer film.
When the melt flow rate of the 4-methyl-1-pentene-based polymer (A1) is within the above-described range, it is easy to extrusion-mold the 4-methyl-1-pentene-based polymer (A1) to have a relatively uniform film thickness.
The melt flow rate (MFR) of the 4-methyl-1-pentene-based polymer (A1) is a value as measured at 230 °C under a load of 2.16 kg according to ASTMD1238.

-Density-

The density of the 4-methyl-1-pentene-based polymer (A1) is preferably from 820 $kg/m^3$ to 870 $kg/m^3$, and more preferably from 830 $kg/m^3$ to 850 $kg/m^3$, from the viewpoint of handleability.
When the density of the 4-methyl-1-pentene-based polymer (A1) is 820 $kg/m^3$ or more, the multilayer film has excellent mechanical strength, and problems such as breakage vulnerability do not tend to occur when the multilayer film is used, for example, as a packaging component or a packaging material. When the density of the 4-methyl-1-pentene-based polymer (A1) is 870 $kg/m^3$ or less, a multilayer film having a high gas permeability can be obtained.
The density of the 4-methyl-1-pentene-based polymer (A1) is a value as measured according to JISK 7112 (the density gradient tube method).

(Method of Producing 4-methyl-1-pentene-based Polymer (A1))

Preferable examples of polymerization catalysts that can be used in the preparation of the 4-methyl-1-pentene-based polymer (A1) include known catalysts, for example, magnesium-bearing titanium catalysts, and metallocene catalysts described in WO 01/53369, WO 01/27124, JPH 3-193796A, or JPH02-41303A. The method used for producing the 4-methyl-1-pentene-based polymer (A1) may be a method described in WO 01/27124, WO 14/050817, or the like.
The thickness of the 4-methyl-1-pentene layer is preferably from 5 μm to 70 μm, more preferably from 5 μm to 50 μm, and still more preferably from 5 μm to 40 μm, and particularly preferably from 5 μm to 30 μm, from the viewpoint of gas permeability.

(1-butene-based Polymer (B1))

The 4-methyl-1-pentene layer may further include a 1-butene-based polymer (B1). The 1-butene-based polymer (B1) may be a polymer including a structural unit derived from 1-butene, a structural unit derived from ethylene, and a structural unit derived from propylene. The amount of structural units derived from 1-butene is from 5 mol% to 100 mol%, assuming that the total amount of structural units derived from 1-butene, structural units derived from ethylene, and structural units derived from propylene is 100 mol%. An amount of structural units derived from 1-butene within the above-described ranges is preferable in terms of interlayer adhesion strength.
In an aspect of the 1-butene-based polymer (B1), the amount of structural units derived from 1-butene is more preferably in the range of from 50 mol% to 100 mol%, and still more preferably from 70 mol% to 90 mol%. An amount of structural units derived from 1-butene within these ranges is particularly preferable in terms of interlayer adhesion strength.
In another aspect of the 1-butene-based polymer (B 1), the amount of structural units derived from 1-butene is more preferably in the range of from 5 mol% to less than 50 mol%, and still more preferably from 5 mol% to 35 mol%. An amount of structural units derived from 1-butene within these ranges is particularly preferable in terms of transparency of the multilayer film.
The amount of structural units derived from ethylene is preferably in the range of from 5 mol% to 50 mol%, more preferably in the range of from 5 mol% to 30 mol%, and still more preferably in the range of from 5 mol% to 20 mol%, assuming that the total amount of structural units derived from 1-butene, structural units derived from ethylene, and structural units derived from propylene is 100 mol%.

The amount of structural units derived from propylene is preferably in the range of from 5 mol% to 90 mol%, more preferably in the range of from 10 mol% to 80 mol%, still more preferably in the range of from 20 mol% to 80 mol%, and particularly preferably in the range of from 20 mol% to 70 mol%, assuming that the total amount of structural units derived from 1-butene, structural units derived from ethylene, and structural units derived from propylene is 100 mol%.

The contents of the respective structural unit species in the 1-butene-based polymer (B 1) can be adjusted, for example, based on the amounts of the respective components added during the polymerization reaction. When the 1-butene-based polymer (B 1) is prepared by polymerization, one of ethylene or propylene may be used, or both of ethylene and propylene may be used together, in which case the ratio between ethylene and propylene is not particularly limited.

The 1-butene-based polymer (B 1) may be, for example, a polymer obtained by polymerization of 1-butene and at least one of ethylene or propylene. In other words, the 1-butene-based polymer (B 1) may be a polymer that does not include a structural unit derived from propylene, or a polymer that does not include a structural unit derived from ethylene.

The total amount of structural units derived from 1-butene, structural units derived from ethylene, and structural units derived from propylene is usually 90 mol% or more, preferably 95 mol% or more, more preferably 98 mol% or more, and particularly preferably 100 mol%, with respect to all structural units.

In other words, the polymer (B 1) may further include a structural unit other than a structural unit derived from 1-butene, ethylene, or propylene, as far as the advantageous effect according to the present disclosure is not impaired. The other structural unit is, for example, an $\alpha$-olefin having 20 or fewer carbon atoms. Examples thereof include 1-pentene, 1-hexene, 1-octene, and 1-decene.

The polymer (B1) preferably satisfies the following condition with respect to melt flow rate (MFR), in addition to the foregoing conditions.

- Melt Flow Rate -

The melt flow rate (MFR) of the polymer (B1) is preferably from 0.1 g/10 min. to 100 g/10 min., and more preferably from 0.5 g/10 min. to 50 g/10 min., from the viewpoint of the formability of the multilayer film and the mechanical properties of the multilayer film.

The melt flow rate (MFR) of the polymer (B1) is a value as measured at 230 °C under a load of 2.16 kg according to the ASTMD 1238.

(Method of Producing 1-butene-based Polymer (B1))

Preferable examples of polymerization catalysts that can be used in the preparation of the polymer (B1) include known catalysts, for example, magnesium-bearing titanium catalysts, and metallocene catalysts described in WO 01/53369, WO 01/27124, JPH 3-193796A, or JPH02-41303A. As the method used for producing the polymer (B1), a method described in WO 2014/042249, WO 2006/057361, or the like may be adopted.

(Tackifier)

The 4-methyl-1-pentene layer may include a tackifier. Examples of the tackifier include liquid polybutene, amorphous polyolefins, aliphatic hydrocarbon resins, aromatic hydrocarbon resins, aliphatic-aromatic-copolymerized hydrocarbon resins, dicyclopentadiene-based hydrocarbon resins, hydrogenated aliphatic hydrocarbon resins, hydrogenated aromatic hydrocarbon resins, hydrogenated aliphatic-aromatic-copolymerized hydrocarbon resins, hydrogenated dicyclopentadiene-based hydrocarbon resins, synthetic terpene-based hydrocarbon resins, terpene-based hydrocarbon resins, hydrogenated terpene-based hydrocarbon resins, coumarone-indene-based hydrocarbon resins, low-molecular-weight styrene-based resins, and rosin-based hydrocarbon resins.

The tackifier may be used singly, or in the form of a mixture of two or more thereof.

A commercial product of the tackifier is, for example, CLEARON (product name) manufactured by Yasuhara Chemical Co., Ltd.

Among the above, liquid polybutene is preferable as the tackifier, from the viewpoint of maintaining a favorable gas permeability of the resultant multilayer film.

The content of the tackifier is preferably from more than 0% by mass to 15% by mass, and more preferably from 0.5% by mass to 10% by mass, with respect to 100 parts by mass of the 4-methyl-1-pentene-based polymer.

When the content of the tackifier is more than 0% by mass, more favorable interlayer adhesion strength can be obtained. When the content of the tackifier is 15% by mass or less, the peel force required at the time of peeling is prevented from becoming excessively large.

The kinetic viscosity of the tackifier at 100 °C is preferably from 0.02 cm$^2$/s to 50 cm$^2$/s. The kinetic viscosity is measured and calculated according to the method specified in JIS K2283.

In the layer including a 4-methyl-1-pentene-based polymer, the content of the 4-methyl-1-pentene-based polymer is preferably from 70 parts by mass to 100 parts by mass, more preferably from 80 parts by mass to 100 parts by mass, and still more preferably from 90 parts by mass to 100 parts by mass.

<Layer That Includes Polyolefin>

The multilayer film according to the present disclosure includes a layer that includes a polyolefin (also referred to as a "polyolefin layer").

Examples of the polyolefin included in the polyolefin layer include polyethylene, polypropylene, and $\alpha$-olefin copolymers.

Examples of the polyethylene include a low-density polyethylene, a linear low-density polyethylene, and a high-density polyethylene.

Examples of the $\alpha$-olefin copolymers include a copolymer of two or more monomers selected from the group consisting of ethylene, propylene, 1-butene, 1-hexene, and 1-octene.

Examples of ethylene-$\alpha$-olefin copolymers include a copolymer of ethylene and at least one monomer selected from the group consisting of propylene, 1-butene, 1-hexene, and 1-octene.

As the polyolefin configuring the polyolefin layer, one polyolefin may be used alone, or two or more polyolefins may be used together.

The polyolefin preferably includes a low-density polyethylene or a linear low-density polyethylene.

The polyolefin preferably includes at least one selected from the group consisting of unsaturated carboxylic acid-modified ethylene-$\alpha$-olefin copolymers, ethylene-unsaturated carboxylic acid-based copolymers, ethylene-vinyl acetate copolymers, silane-modified ethylene-vinyl acetate copolymers, ionomers of unsaturated carboxylic acid-modified ethylene-$\alpha$-olefin copolymers, and ionomers of ethylene-unsaturated carboxylic acid-based copolymers, and more preferably includes an ethylene-unsaturated carboxylic acid-based copolymer or an ionomer thereof.

(Unsaturated Carboxylic Acid-Modified Ethylene-$\alpha$-Olefin Copolymer)

The ethylene-$\alpha$-olefin copolymer to be used in the unsaturated carboxylic acid-modified ethylene-$\alpha$-olefin copolymer may be a copolymer of ethylene and an $\alpha$-olefin having from 3 to 20 carbon atoms.

Examples of the $\alpha$-olefin having from 3 to 20 carbon atoms include propylene, 1-butene, 1-hexene, 1-octene, 1-decene, 1-dodecene, 1-tetradecene, 1-hexadecene, 1-octadecene, and 1-eicosene.

Among them, the $\alpha$-olefin having from 3 to 20 carbon atoms is preferably propylene, 1-butene, 1-hexene, or 1-octene, and more preferably 1-butene, 1-hexene, or 1-octene, from the viewpoint of adhesion strength.

The molar ratio between ethylene and $\alpha$-olefin(s) in the ethylene-$\alpha$-olefin copolymer is preferably in the range of from 45/55 to 95/5.

In the present disclosure, scope of the unsaturated carboxylic acid used for the modification include unsaturated carboxylic acids and derivatives thereof.

An unsaturated carboxylic acid or a derivative thereof is used as the unsaturated carboxylic acid for use in the modification, and examples thereof include unsaturated carboxylic acids such as maleic acid, fumaric acid, tetrahydrophthalic acid, itaconic acid, citraconic acid, crotonic acid, isocrotonic acid, nadic acid (registered trademark; endocisbicyclo[2.2.1]hept-5-ene-2,3-dicarboxylic acid), acrylic acid, and methacrylic acid, or derivatives thereof.

Examples of the derivatives of unsaturated carboxylic acids include acid anhydrides, imides, amides, and esters of unsaturated carboxylic acids.

Specific examples of the derivatives of unsaturated carboxylic acids include maleimide, maleic anhydride, citraconic anhydride, monomethyl maleate, and glycidyl maleate.

Among the above, an unsaturated carboxylic acid or an acid anhydride thereof is preferable, and maleic acid, maleic anhydride, nadic acid, and nadic anhydride are more preferable, as the unsaturated carboxylic acid for use in the modification.

The method used for performing modification with an unsaturated carboxylic acid is, for example, a method including performing modification by means of a graft reaction of the unsaturated carboxylic acid.

Known methods may be used as the method used for producing a modified product by means of performing graft copolymerization using an unsaturated carboxylic acid or a derivative thereof as a graft monomer.

For example, a melt modification method including melting an ethylene-$\alpha$-olefin copolymer, adding a graft monomer, and performing graft copolymerization, a solution modification method including dissolving an ethylene-$\alpha$-olefin copolymer in a solvent, adding a graft monomer, and performing graft copolymerization, and the like can be used.

When the graft copolymerization is performed, the reaction is preferably performed in the presence of a radical initiator, from the viewpoint of efficiency.

The content of the radical initiator may be in the range of from 0.001 parts by mass to 2 parts by mass with respect to 100 parts by mass of the ethylene-$\alpha$-olefin copolymer, which is the base polymer.

Examples of the radical initiator include organic peroxides such as dicumyl peroxide, di-tert-butyl peroxide, 2,5-dimethyl-2,5-di(tert-butylperoxy)hexyne-3,2,5-dimethyl-2,5-di(tert-butylperoxy)hexane, and 1,4-bis(tert-butylperoxyisopropyl)benzene.

In the unsaturated carboxylic acid-modified ethylene-$\alpha$-olefin copolymer in the present disclosure, the modification amount may be from 0.01% by mass to 10% by mass, preferably from 0.1% by mass to 5% by mass, and more preferably from 1% by mass to 5% by mass, with respect to the total amount of the unsaturated carboxylic acid-modified ethylene-$\alpha$-olefin copolymer.

When the modification amount is within the above-described ranges, the multilayer film has a more favorable interlayer adhesion strength between the 4-methyl-1-pentene layer and the polyolefin layer.

The melt flow rate (MFR) of the unsaturated carboxylic acid-modified ethylene-$\alpha$-olefin copolymer as measured according to the ASTM D1238 under conditions of a temperature of 190 °C and a load of 2.16 kg is preferably in the range of from 0.05 g/10 min. to 200 g/10 min., and more preferably in the range of from 0.1 g/10 min. to 100 g/10 min.

The unsaturated carboxylic acid-modified ethylene-$\alpha$-olefin copolymer may have a crystallinity as measured with X-rays of 30% or less.

(Ethylene-Unsaturated Carboxylic Acid-Based Copolymer)

The ethylene-unsaturated carboxylic acid-based copolymer is a copolymer that includes a structural unit derived from ethylene and a structural unit derived from an unsaturated carboxylic acid.

The ethylene-unsaturated carboxylic acid-based copolymer in the present disclosure may be a copolymer of ethylene and an unsaturated carboxylic acid, a copolymer of ethylene, an unsaturated carboxylic acid, and another monomer, or a polymer obtained by modifying an ethylene polymer with an unsaturated carboxylic acid.

Examples of the ethylene-unsaturated carboxylic acid-based copolymer include ethylene-unsaturated carboxylic acid binary copolymers and ionomers thereof, ethylene-unsaturated carboxylic acid-unsaturated carboxylic acid ester ternary copolymers and ionomers thereof, and ethylene-unsaturated carboxylic acid ester binary copolymers. From the viewpoint of interlayer adhesion strength and heat-seal strength, ethylene-unsaturated carboxylic acid-unsaturated carboxylic acid ester ternary copolymers and ionomers thereof are preferable.

The ethylene-unsaturated carboxylic acid-based copolymer can be obtained, for example, by allowing ethylene and an unsaturated carboxylic acid to undergo a radical polymerization reaction at high temperature and high pressure in the presence of an organic peroxide or oxygen.

Examples of the unsaturated carboxylic acid include ethyl acrylate, methacrylic acid, methyl methacrylate, vinyl acetate, and vinyl chloride.

Among them, at least one selected from the group consisting of ethyl acrylate, methacrylic acid, methyl methacrylate, and vinyl acetate is preferable, and at least one selected from the group consisting of ethyl acrylate, methacrylic acid, and methyl methacrylate is more preferable, from the viewpoint of achieving excellent heat resistance and enabling processing at high temperatures.

A copolymer of ethylene and ethyl acrylate (also referred to as "EEA" in the present disclosure) can be used as the ethylene-unsaturated carboxylic acid-based copolymer.

In the EEA, the content of ethyl acrylate may be from 10% by mass to 40% by mass, and preferably from 15% by mass to 30% by mass, from the viewpoint of formability and interlayer adhesion strength.

The melt flow rate (as measured according to the ASTM D1238 at a temperature of 190 °C under a load of 2.16 kg) thereof may be from 5 g/10 min. to 50 g/10 min., and preferably from 10 g/10 min. to 30 g/10 min., from the viewpoint of formability and interlayer adhesion strength.

A copolymer of ethylene and methacrylic acid (also referred to as "EMAA" in the present disclosure) can be used as the ethylene-unsaturated carboxylic acid-based copolymer.

In the ethylene-methacrylic acid copolymer, the content of methacrylic acid may be from 10% by mass to 40% by mass, and preferably from 3% by mass to 10% by mass, from the viewpoint of formability and interlayer adhesion strength.

The melt flow rate (as measured according to the ASTM D1238 at a temperature of 190 °C under a load of 2.16 kg) thereof may be from 5 g/10 min. to 100 g/10 min., and preferably from 10 g/10 min. to 80 g/10 min., from the viewpoint of formability and interlayer adhesion strength.

A commercial product thereof is, for example, NUCREL (registered trademark) manufactured by Dow-Mitsui Polychemicals Co., Ltd.

(Ethylene-Vinyl acetate Copolymer and Silane-modified Ethylene-Vinyl acetate Copolymer)

An ethylene-vinyl acetate copolymer (also referred to as "EVA") is a copolymer of ethylene and vinyl acetate.

A silane-modified ethylene-vinyl acetate copolymer (also referred to as "silane-modified EVA") is a compound obtained by modifying EVA.

The content of vinyl acetate in the EVA is preferably from 10% by mass to 50% by mass, from the viewpoint of adhesion strength. The EVA has a density of preferably from 930 kg/m³ to 980 kg/m³. The EVA has a melt flow rate (MFR) at 190 °C under a load of 2.16 kg of preferably from 0.8 g/10 min. to 30 g/10 min.

As the ethylene-vinyl acetate copolymer, those produced using known methods may be used, as appropriate, or commercially available ethylene-vinyl acetate copolymers may be used.

The silane compound used for modifying the EVA is preferably an organic silicon compound having, in a molecule thereof, one or more unsaturated hydrocarbon groups and one or more alkoxy groups.

Examples of the unsaturated hydrocarbon groups include a vinyl group, an allyl group, and a (meth)acrylic group, and examples of the alkoxy groups include a methoxy group, an ethoxy group, and a butoxy group.

Examples of the silane compound include vinyl trimethoxy silane and vinyl triethoxy silane, which have a vinyl group as an unsaturated hydrocarbon group and a methoxy or ethoxy group as an alkoxy group.

The content of the silane compound in the silane-modified EVA is preferably from 0.01 parts by mass to 5.0 parts by mass with respect to 100 parts by mass of EVA.

The silane-modified EVA in the present disclosure may be a compound obtained by graft-modifying EVA with a silane compound.

For example, the silane-modified EVA can be obtained by adding a silane compound and a radical generator to EVA in an extruder, melt-kneading the resultant mixture at a temperature equal to or higher than the heat decomposition initiation temperature of the radical generator for a time period, and pelletizing the kneaded material.

The radical generator is a compound that generates a free radical when the compound is decomposed by heating, and the radical generator is used as a reaction initiator for allowing a silane compound to chemically bind to EVA. A radical generator having a half-life of 3 minutes or less at a temperature that is equal to or higher than the melting point of the EVA but not higher than 150 °C, is particularly preferable.

Examples of the radical generator include peroxides such as benzoyl peroxide, lauroyl peroxide, t-butyl peracetate, t-butyl peroxy-2-ethylhexanoate, t-butyl peroxyisobutyrate, and dicumylperoxide.

The content of the radical generator may appropriately be adjusted in accordance with the type of EVA and the amount of the silane compound used. For example, the content of the radical generator is preferably from 0.01 parts by mass to 5.0 parts by mass with respect to 100 parts by mass of EVA.

A reaction inhibitor such as mercaptan may be used to inhibit polymerization between molecules of the silane compound.

The polyolefin in the present disclosure has a D hardness of preferably from 20 to 80, more preferably from 30 to 70, and still more preferably from 30 to 55, from the viewpoint of increasing the interlayer adhesion strength.

The polyolefin layer preferably includes a slip agent. The slip agent is preferably an amide of a saturated or unsaturated fatty acid such as lauric acid, palmitic acid, oleic acid, stearic acid, erucic acid, or behenic acid, or a bisamide of any of these saturated or unsaturated fatty acids. Among them, erucic amide and ethylene bisstearamide are more preferable.

The content of the slip agent is preferably from 0.01 parts by mass to 5 parts by mass with respect to 100 parts by mass of polyolefin contained in the polyolefin layer.

<Heat-seal Strength between Polyolefin Layers>

The multilayer film according to the present disclosure exhibits a heat-seal strength (also referred to as the "heat-seal strength between polyolefin layers" in the present disclosure) of from 3 N/15 mm to 15 N/15 mm in a case in which two of the layers that include a polyolefin are brought into contact with each other and heat-sealed at 120 °C.

Due to the heal-seal strength between polyolefin layers being 3 N/15 mm or more, the multilayer film according to the present disclosure has excellent heat-sealability at low temperatures.

From the foregoing viewpoint, the heal-seal strength between polyolefin layers is preferably 3 N/15 mm or more.

The heat-seal strength is measured according to the following method. Specifically, two multilayer film sheets including the layer that includes a 4-methyl-1-pentene-based polymer and the layer that includes a polyolefin are prepared. The polyolefin layers of the multilayer films are allowed to face each other, and heat-sealed at a heat-sealing temperature of 120 °C, a pressure of 0.2 MPa, and a seal bar width of 5 mm for a sealing time of 1 second, followed by heat dissipation. Then, a 15-mm-wide test piece is cut out of the test material obtained by the heat-sealing, and a peel strength at the time of peeling the heat-sealed portion at a cross-head speed of 300 mm/min. is measured, and the value obtained is taken as the heat-seal strength.

The thickness of the polyolefin layer is preferably from 1 $\mu$m to 15 $\mu$m, more preferably from 3 $\mu$m to 15 $\mu$m, and still more preferably from 5 $\mu$m to 10 $\mu$m, from the viewpoint of maintaining the balance between the gas permeability and interlayer adhesion strength of the multilayer film.

The shape of the polyolefin layer is not particularly limited, and it is preferable that irregularities are formed on the surface of the polyolefin layer. For example, it is preferable that the surface of the polyolefin layer includes embossing.

When irregularities are formed on the surface of the polyolefin layer, handleability can be improved. Further, it is also possible to impart a texture to the multilayer film.

<Interlayer Adhesion Strength>

The multilayer film according to the present disclosure has an interlayer adhesion strength between the 4-methyl-1-pentene layer and the polyolefin layer of 0.5 N/15 mm or more. Due to the interlayer adhesion strength being 0.5 N/15 mm or more, the multilayer film according to the present disclosure has an excellent heat-sealability.

From the foregoing viewpoint, the interlayer adhesion strength between the 4-methyl-1-pentene layer and the polyolefin layer is preferably 1.0 N/15 mm or more, more preferably 1.5 N/15 mm or more, and still more preferably 2.0 N/15 mm or more.

The upper limit of the interlayer adhesion strength between the 4-methyl-1-pentene layer and the polyolefin layer is not particularly limited.

For example, in the multilayer film according to the present disclosure, the interlayer adhesion strength between the 4-methyl-1-pentene layer and the polyolefin layer may be from 0.5 to 20.0 N/15 mm or less, and may be from 0.6 to 10.0 N/15 mm or less.

The interlayer adhesion strength is measured according to the following method. Specifically, a 15 mm-wide test piece is cut out of the multilayer film (laminated body), and the interlayer adhesion strength is measured according to JIS Z1707: 1997, using a tensile tester (for example, AUTOGRAPH AG-X manufactured by Shimadzu Corporation). The measurement of the interlayer adhesion strength is performed by measuring the interlayer adhesion strength between the 4-methyl-1-pentene layer and the polyolefin layer of the test piece, under conditions of a distance between chucks of 90 mm and a pulling rate of 500 mm/min.

The multilayer film according to the present disclosure may be an oxygen permeable multilayer film or a carbon dioxide permeable multilayer film. An oxygen permeable multilayer film refers to a multilayer film having an oxygen permeability of 1000 mL/m$^2$·day·atm or more. A carbon dioxide permeable multilayer film refers to a multilayer film having a carbon dioxide permeability of 3000 mL/m$^2$·day·atm or more.

[Properties of Multilayer Film]

-Gas Permeability-

The moisture permeability (according to JIS Z0208 at a test temperature of 40 °C and a test humidity of 90%) of the multilayer film according to the present disclosure is preferably from 10 g/m$^2$·day to 80 g/m$^2$·day, and more preferably from 25 g/m$^2$·day to 70 g/m$^2$·day, from the viewpoint of preventing mold or the like during storage of a fruit or a vegetable, a flower or an ornamental plant, a seed or seedling, a microorganism, or the like.

The oxygen permeability of the multilayer film according to the present disclosure is not particularly limited. The oxygen permeability of the multilayer film according to the present disclosure is preferably from 1000 mL/m$^2$·day·atm to 100,000 mL/m$^2$·day·atm, more preferably from 2500 mL/m$^2$·day·atm to 85,000 mL/m$^2$·day·atm, still more preferably from 5000 mL/m$^2$·day·atm to 80,000 mL/m$^2$·day·atm, particularly preferably from 10,000 mL/m$^2$·day·atm to 50,000 mL/m$^2$·day·atm, and still further preferably from 11,000 mL/m$^2$·day·atm to 50,000 mL/m$^2$·day·atm, from the viewpoint of storing a fruit or a vegetable, a flower or an ornamental plant, a microorganism, or the like for a long period of time.

A combination of an upper limit value for the oxygen permeability and a lower limit value for the oxygen permeability can be selected, as desired. For example, the oxygen permeability of the multilayer film according to the present disclosure is also preferably from 11,000 mL/m$^2$·day·atm to 100,000 mL/m$^2$·day·atm, more preferably from 11,000 mL/m$^2$·day·atm to 85,000 mL/m$^2$·day·atm, and still more preferably from 11,000 mL/m$^2$·day·atm to 80,000 mL/m$^2$·day·atm.

The carbon dioxide permeability of the multilayer film according to the present disclosure is not particularly limited. The carbon dioxide permeability of the multilayer film according to the present disclosure is preferably from 8000 mL/m$^2$·day·atm to 250,000 mL/m$^2$·day·atm, more preferably from 25,000 mL/m$^2$·day·atm to 230,000 mL/m$^2$·day·atm, still more preferably from 50,000 mL/m$^2$·day·atm to 200,000 mL/m$^2$·day·atm, and particularly preferably from 50,000 mL/m$^2$·day·atm to 150,000 mL/m$^2$·day·atm, from the viewpoint of storing a fruit or a vegetable, a flower or an ornamental plant, a microorganism, or the like for a long period of time.

A combination of an upper limit value for the carbon dioxide permeability and a lower limit value for the carbon dioxide permeability can be selected, as desired. For example, the carbon dioxide permeability of the multilayer film according to the present disclosure is also preferably from 25,000 mL/m$^2$·day·atm to 250,000 mL/m$^2$·day·atm, more preferably from 25,000 mL/m$^2$·day·atm to 230,000 mL/m$^2$·day·atm, still more preferably from 25,000 mL/m$^2$·day·atm to 20,000 mL/m$^2$·day·atm, and particularly preferably from 25,000 mL/m$^2$·day·atm to 15,000 mL/m$^2$·day·atm.

The hydrogen permeability of the multilayer film according to the present disclosure is not particularly limited. The hydrogen permeability of the multilayer film according to the present disclosure is preferably from 10,000 mL/m$^2$·day·atm to 300,000 mL/m$^2$·day·atm, more preferably from 50,000 mL/m$^2$·day·atm to 230,000 mL/m$^2$·day·atm, still more preferably from 50,000 mL/m$^2$·day·atm to 200,000 mL/m$^2$·day·atm, and particularly preferably from 50,000 mL/m$^2$·day·atm to 150,000 mL/m$^2$·day·atm, from the viewpoint of favorable usability of the multilayer film in a hydrogen battery-related members such as a hydrogen separation membrane or a hydrogen permeable membrane.

It is preferable that the multilayer film according to the present disclosure has an oxygen permeability of 2500 mL/m$^2$·day·atm to 85,000 mL/m$^2$·day·atm, a carbon dioxide permeability of from 8000 mL/m$^2$·day·atm to 250,000 mL/m$^2$·day·atm, and a hydrogen permeability of from 10,000 mL/m$^2$·day·atm to 300,000 mL/m$^2$·day·atm.

The oxygen permeability (unit: mL/m$^2$·day·atm), carbon dioxide permeability (unit: mL/m$^2$·day·atm), and hydrogen permeability (unit: mL/m$^2$·day·atm) of the multilayer film can be measured according to, for example, JIS K7126-1 (the differential pressure method) or JIS K7126-2 (the equal-pressure method).

When the oxygen permeability and carbon dioxide permeability of the multilayer film are measured according to JIS K7126-1 (the differential pressure method), the measurement is carried out by the following method. A piece obtained by cutting the multilayer film into a shape having a width of 30 mm and a length of 30 mm is used as a test piece.

The measurement is carried out according to JIS K7126-1 at a test temperature of 23 °C and a test humidity of 0% RH with a measurement area of the multilayer film set to 5 cm$^2$, using a differential pressure method gas permeability measurement apparatus (for example, a differential pressure method gas permeability measurement apparatus manufactured by Toyo Seiki Seisaku-sho Ltd.) Two sheets of aluminum masks provided with a pressure-sensitive adhesive and manufactured by Modern Control, each of which has a hole having a diameter of 25 mm at a central part thereof, are prepared, the two mask sheets are laminated while the multilayer film to be measured is sandwiched therebetween, and the measurement area in the multilayer film is adjusted. More specifically, the multilayer film is disposed such that the holes at the central portions of the two mask sheets overlap each other.

When the hydrogen permeability (unit: mL/m$^2$·day·atm) of the multilayer film is measured according to JIS K7126-2, the hydrogen permeability is measured by the following method.

Measurement Instrument: equal-pressure method gas permeability measurement instrument (GTR-10XFKS manufactured by GTE TEC Corporation)
Test Piece: test piece having a diameter of 50 mm
Detector: gas chromatographic detection
Test Gas: hydrogen
Measurement Temperature: 23 °C
Permeation Area: area having a diameter of 35 mm

The moisture permeability (unit: g/m$^2$·day) of the multilayer film is measured by the following method. Specifically, the moisture permeability is measured according to JIS Z0208 the equal-pressure method (the weight method using a cup) under conditions of a test temperature of 40 °C and a test humidity of 90% RH with a measurement area of the multilayer film set to 50 cm$^2$, using anhydrous calcium chloride as a desiccant. The multilayer film is placed in a constant-temperature constant-humidity apparatus, and the mass is measured (at a unit of 0.1 mg) at 24 hour intervals for 10 days, which is an approximate period for achieving an almost constant increased mass, and the moisture permeability is calculated therefrom.

The total haze of the multilayer film according to the present disclosure is from 0.1% to 10.0%, and preferably from 0.1% to 9.5%.

When the total haze is within the above-described ranges, high transparency can be obtained, and visibility of the content is heightened in a case in which the multilayer film is used as a packaging component or a packaging material. Therefore, a total haze within the above-described ranges is preferable.

[Method of Producing Multilayer Film]

The method of producing a multilayer film according to the present disclosure is a method of producing the multilayer film of the present disclosure.

The method of producing a multilayer film according to the present disclosure is not particularly limited, and is preferably a method in which the layers included as constituent elements of the multilayer film of the present disclosure mix with each other at or around the interface therebetween and become adhered thereby, to form a multilayer film.

Examples of such a method include a co-extrusion method including laminating molten resins and a heat fusing method including heat-fusing an already-formed resin multilayer film.

Among these methods, the co-extrusion method including laminating molten resins is more preferable from the viewpoint that the co-extrusion method can form a multilayer film having a higher interlayer adhesion strength between respective layers in which separation between layers is less likely to occur.

The method of producing a multilayer film according to the present disclosure preferably includes a step of laminating a layer that includes a polyolefin onto a base material that includes a 4-methyl-1-pentene-based polymer, using the direct lamination method. Inclusion of this step makes it possible to obtain a multilayer film in which the 4-methyl-1-pentene layer and the polyolefin layer contact each other at at least a portion thereof.

The direct lamination method is a method including laminating the 4-methyl-1-pentene layer and the polyolefin layer while allowing the 4-methyl-1-pentene layer to directly contact the polyolefin layer.

Specifically, the direct lamination method may be, for example, a method including directly adhering, to each other, (i) a raw material for the polyolefin layer extruded from an extruder and (ii) a film including 4-methyl-1-pentene that has been conveyed, thereby achieving lamination.

Surface treatment is preferably performed, for example, on the film including 4-methyl-1-pentene, before the 4-methyl-1-pentene layer and the polyolefin layer are laminated. Performing surface treatment makes it possible to obtain a more improved interlayer adhesion strength. Examples of the surface treatment include corona treatment and ozone treatment. The corona treatment can be conducted using, for example, a Corona Master (for example, PS-10S manufactured by Shinko Electric & Instrumentation Co., Ltd.). The ozone treatment can be conducted using a UV ozone cleaner (for example, UV42 manufactured by Nihon Laser Denshi Kabushiki Kaisha).

Examples of the surface treatment also include plasma treatment, UV treatment, and flame treatment.

In the multilayer film according to the present disclosure, it is preferable that at least one side of the layer including a 4-methyl-1-pentene-based polymer has been subjected to at least one treatment selected from the group consisting of plasma treatment, corona treatment, ozone treatment, UV treatment, and flame treatment.

The multilayer film according to the present disclosure may be a multilayer film obtained by extrusion-laminating a layer including a polyolefin onto a layer including a 4-methyl-1-pentene-based polymer.

The method of producing a multilayer film according to the present disclosure may be, specifically, a method of producing a multilayer film including:

a treatment step of performing a surface treatment on at least one side of a base material that includes a 4-methyl-1-pentene-based polymer; and
a lamination step of laminating a polyolefin onto the side of the base material that has been subjected to the surface treatment, to produce a multilayer film,
wherein, in the multilayer film, an inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

<Treatment Step>

The treatment step is a step of performing a surface treatment on at least one side of a base material that includes a 4-methyl-1-pentene-based polymer. Details, such as specific modes and preferable aspects, of the surface treatment are as described above.

The surface treatment preferably includes at least one treatment selected from the group consisting of plasma treatment, corona treatment, ozone treatment, UV treatment, and flame treatment.

<Lamination Step>

The lamination step is a step of laminating a polyolefin onto the side of the base material that has been subjected to the surface treatment. Details, such as specific modes and preferable aspects, of the method of laminating a polyolefin are as described above.

The lamination step is preferably a step of performing an ozone treatment on a polyolefin, bringing a side of the polyolefin that has been subjected to the ozone treatment and a side of the base material that has been subjected to the surface treatment into contact with each other, and laminating the polyolefin onto the side of the base material that has been subjected to the surface treatment.

The lamination step is preferably a step of laminating a polyolefin onto a base material including a 4-methyl-1-pentene-based polymer by means of extrusion lamination.

[Use of Multilayer Film]

The multilayer film according to the present disclosure can widely be used, for example, as a tape, a pressure-adhesive tape, a masking tape, a masking multilayer film, a temporary attaching multilayer film, a packaging multilayer film for maintaining freshness, a plastic envelope, an easy-open packaging bag, a multilayer film for automatic packaging, a shopping bag, a standing bag, a multilayer film container for liquid, a transparent packaging box, a building material, a multilayer film for adhesion, a multilayer film for agriculture, a food packaging material, a fruit packaging material, a packaging material for a flower or an ornamental plant, a packaging material for an electronic part, a packaging material for a mechanical part, a crop packaging material, a packaging material for an aquatic product such as fish or seafood, a medical multilayer film, a medical tape, a pack for cultivating plant cells, a pack for storing and growing a seed or a seedling, a pack for cultivating animal cells, a carbon dioxide separation membrane, a hydrogen separation membrane, or an oxygen enrichment membrane.

<<Container>>

The container according to the present disclosure is a container that includes the multilayer film of the present disclosure, and that is configured to be sealed by mutually heat-sealing the layers that include a polyolefin included in the multilayer film(s).

The container according to the present disclosure is a container produced using the multilayer film according to the present disclosure, and the layer that includes a polyolefin and that is included in the multilayer film functions as a heat-seal layer.

<<Pack for Cell Culture>

The pack for cell culture according to the present disclosure includes the multilayer film according to the present disclosure. The multilayer film according to the present disclosure can favorably be used as a pack for culturing cells.

Embodiments according to the present disclosure also include the following aspects.

<1> A multilayer film, including a layer that includes a 4-methyl-1-pentene-based polymer, and a layer that includes a polyolefin, wherein the layer that includes a 4-methyl-1-pentene-based polymer and the layer that includes a polyolefin contact each other at at least a portion thereof, an inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

<2> The multilayer film of <1>, wherein the polyolefin includes at least one selected from the group consisting of an unsaturated carboxylic acid modified ethylene-$\alpha$-olefin copolymer, an ethylene-unsaturated carboxylic acid-based copolymer, an ethylene-vinyl acetate copolymer, a silane modified ethylene-vinyl acetate copolymer, an ionomer of an unsaturated carboxylic acid modified ethylene-$\alpha$-olefin copolymer, and an ionomer of an ethylene-unsaturated carboxylic acid-based copolymer.

<3> The multilayer film of <1> or <2>, wherein the layer that includes a 4-methyl-1-pentene-based polymer has a thickness of from 5 $\mu$m to 50 $\mu$m.

<4> The multilayer film of any one of <1> to <3>, wherein the layer that includes a polyolefin has a thickness of from 3 $\mu$m to 15 $\mu$m.

<5> The multilayer film of any one of <1> to <4>, wherein the multilayer film is an oxygen permeable multilayer film or a carbon dioxide permeable multilayer film.

<6> The multilayer film of any one of <1> to <5>, wherein the multilayer film has an oxygen permeability of from 1000 mL/m$^2$·day·atm to 100,000 mL/m$^2$·day·atm, and a carbon dioxide permeability of from 8000 mL/m$^2$·day·atm to 250,000 mL/m$^2$·day·atm.

<7> The multilayer film of any one of <1> to <6>, wherein the multilayer film has a hydrogen permeability of from 10,000 mL/m$^2$·day·atm to 300,000 mL/m$^2$·day·atm.

<8> The multilayer film of any one of <1> to <7>, wherein the multilayer film has a moisture permeability of from 10 g/m$^2$·day to 80 g/m$^2$·day.

<9> The multilayer film of any one of <1> to <8>, wherein irregularities are formed on a surface of the layer that includes a polyolefin.

<10> A pack for cell culture, including the multilayer film of any one of <1> to <9>.

EXAMPLES

The present disclosure is more specifically illustrated below by reference to examples. However, the present disclosure is not limited to the following examples as long as the gist of the present disclosure is retained.

In the examples, the following materials were used.

[4-methyl-1-pentene-based polymer]

MP1: F.O.R. WRAP (4-methyl-1-pentene-based polymer film, manufactured by Riken Fabro Corporation)

* MP1 has a content of 4-methyl-1-pentene-based polymer of from 70% by mass to 100% by mass.

MP2: A 4-methyl-1-pentene-based polymer film produced according to the following method.

* MP2 has a content of 4-methyl-1-pentene-based polymer of 100% by mass.

(Preparation of MP2 Film)

TPX MX002O was charged into all single screw extruders of the following threetypes/layers T-die molding machine, and a monolayer film having a thickness of 50 $\mu$m was obtained at a processing temperature of 260 °C, a chill roll temperature of 25 °C, and a haul-off velocity of 10 m/min.

- Extruder: 350 mm-wide three-types/layers T-die molding machine equipped with a 30 mm-diameter single screw extruder, a 40 mm-diameter single screw extruder, and a 30 mm-diameter single screw extruder
- TPX MX002O: 4-methyl-1-pentene-based polymer (manufactured by Mitsui Chemicals, Inc. and having a MFR of 23 g/10 min. (at 260 °C))

MP3: A 4-methyl-1-pentene-based polymer film prepared by the following method.

* MP3 has a content of 4-methyl-1-pentene-based polymer of from 90% by mass, and a content of polybutene-1 of 10% by mass.

(Preparation of MP3 Film)

A monolayer film having a thickness of 30 $\mu$m was obtained by the same method as that in the preparation of the MP2 film, except that a mixture obtained by dry-blending 90% by mass of TPX MX002O and 10% by mass of TAFMER BL2000 was used, and that the haul-off velocity was set to 16 m/min.

- TAFMER BL2000: Polybutene-1 (manufactured by Mitsui Chemicals, Inc., having a MFR of 1.0 g/10 min. (at 190 °C))

[Polyolefin]

PO1: NUCREL AN4228C (ethylene-unsaturated carboxylic acid-based copolymer, manufactured by Dow-Mitsui Polychemicals Co., Ltd.)
PO2: MIRASON 16P (low-density polyethylene, manufactured by Dow-Mitsui Polychemicals Co., Ltd.)

Examples and Comparative Examples are described in detail below. In the Examples and the Comparative Examples, corona treatment was performed on the 4-methyl-1-pentene-based polymer film. Ozone treatment was performed on the polyolefin.

[Multilayer Film]

<Example 1>

The polyolefin was melt-extruded onto MP1 [the 4-methyl-1-pentene-based polymer film] according to the direct lamination method using an extrusion laminator, and a multilayer film was produced under the following production conditions.

•• Processing Conditions ••

Extrusion Laminator: 65 mm-diameter extruder having a T-die opening width of 500 mm
Processing Velocity: 80 m/min.
Extrusion Resin Temperature: from 270 °C to 330 °C (as the resin temperature directly below the T-die, as measured by a contact-type thermometer)
Air Gap: 120 mm
Nip Pressure: 2 kgf
Corona Treatment: output (kW) indicated in Table 1

<Examples 2 and 4>

A multilayer film was obtained in the same manner as that in Example 1, except that the output of the corona treatment was changed to the output value (kW) indicated in Table 1, and the thickness of the polyolefin layer was changed to the thickness indicated in Table 1 by adjusting the processing velocity.

<Example 3>

A multilayer film was obtained in the same manner as that in Example 2, except that ozone treatment, in addition to the corona treatment, was performed as a surface treatment.

<Example 5>

A multilayer film was obtained in the same manner as that in Example 4, except that ozone treatment, in addition to the corona treatment, was performed as a surface treatment.

<Examples 6 to 8>

A multilayer film was obtained in the same manner as that in Example 5, except that the kind of the 4-methyl-1-pentene-based polymer was changed as indicated in Table 1, and that the thickness of the polyolefin layer was changed to the thickness indicated in Table 1 by adjusting the processing velocity.

<Comparative Example 1>

Two MP1 sheets were superposed one on the other, and subjected to heat-sealing. However, the two MP1 sheets did not adhere to each other, and heat-sealing could not be achieved.

<Comparative Example 2>

Production of a multilayer film was tried in the same manner as that in Example 1, except that PO1 [polyolefin] was replaced by PO2 [polyolefin], and that the corona treatment was not performed. However, MP1 and PO2 did not adhere to each other, and a multilayer film could not be obtained.

<Comparative Example 3>

A multilayer film was obtained in the same manner as that in Comparative Example 2, except that the corona treatment and the ozone treatment were performed as surface treatments.

[Evaluations]

Evaluation of the multilayer films of Examples and Comparative Examples was conducted by measuring the interlayer adhesion strength between the 4-methyl-1-pentene layer and the polyolefin layer, the heat-seal strength between the polyolefin layers, the oxygen permeability, and the carbon dioxide permeability, according to the foregoing methods.

When a multilayer film has a carbon dioxide permeability of 70,000 mL/m$^2$·day·atm or more and an oxygen permeability of 20,000 mL/m$^2$·day·atm or more, the multilayer film is considered to have excellent gas permeability.

The heat-seal strength and interlayer adhesion strength of the multilayer film were also measured. The method used for measuring the heat-seal strength was as described below.

Two multilayer film sheets including the layer that includes a 4-methyl-1-pentene-based polymer and the layer that includes a polyolefin were prepared. The polyolefin layers of the multilayer films were allowed to face each other, and heat-sealed at a heat-sealing temperature of 120 °C, a pressure of 0.2 MPa, and a seal bar width of 5 mm for a sealing time of 1 second, followed by heat dissipation. Then, a 15-mm wide test piece was cut out of the test material obtained by the heat-sealing, and a peel strength at the time of peeling the heat-sealed portion at a cross-head speed of 300 mm/min. was measured, and the value obtained was taken as the heat-seal strength.

The method used for measuring the interlayer adhesion strength was as follows.

A 15 mm-wide test piece was cut out of the multilayer film, and the interlayer adhesion strength was measured according to JIS Z1707:1997, using a tensile tester (AUTOGRAPH AG-X manufactured by Shimadzu Corporation). The measurement of the interlayer adhesion strength was performed by measuring the interlayer adhesion strength between the 4-methyl-1-pentene layer and the polyolefin layer of the test piece, under conditions of a distance between chucks of 90 mm and a pulling rate of 500 mm/min.

The results are shown in Table 1. In Example 5, the hydrogen permeability was 109,000 mL/m$^2$·day·atm, and the moisture permeability was 57 g/m$^2$·day. This indicates that the hydrogen permeability and the moisture permeability in Example 5 were excellent.

The hydrogen permeability and the moisture permeability were measured according to the above-described methods.

Table 1

| | | Example 1 | Example 2 | Example 3 | Example 4 | Example 5 | Example 6 | Example 7 | Example 8 | Comparative Example 1 | Comparative Example 2 | Comparative Example 3 |
|---|---|---|---|---|---|---|---|---|---|---|---|---|
| 4-methyl-1-pentene layer | Kind | MP1 | MP1 | MP1 | MP1 | MP1 | MP2 | MP2 | MP3 | MP1 | MP1 | MP1 |
| | Thickness [$\mu$m] | 30 | 30 | 30 | 30 | 30 | 50 | 50 | 30 | 30 | 30 | 30 |
| Polyolefin layer | Kind | PO1 | PO1 | PO1 | PO1 | PO1 | PO1 | PO1 | PO1 | - | PO2 | PO2 |
| | Thickness [$\mu$m] | 5 | 5 | 5 | 10 | 10 | 5 | 10 | 5 | - | 5 | 5 |
| Surface treatment | Corona treatment | 2 kW | 6kW | 6kW | 6kW | 6 kW | 6 kW | 6kW | 6 kW | - | - | 6 kW |
| | Ozone treatment | - | - | Performed | - | performed | performed | performed | performed | - | - | performed |
| Interlayer Strength[N/15mm] | | 2.4 | 2.6 | 4.3 | 0.6 | 2.3 | 3.2 | 3.3 | 4.1 | - | Interlayer separation | Interlayer separation |
| Heat-seal strength between polyolefin layers [N/15mm] | 120 °C | 4.3 | 5.2 | 5.9 | 4.2 | 7.2 | 4.1 | 4.5 | 5.5 | 0.1 or less | - | - |
| | 140 °C | 4.6 | 5 | 6.6 | 6 | 7.8 | - | - | - | 0.1 or less | - | - |
| | 160 °C | 3.4 | 4.3 | 6 | 3.3 | 5.9 | - | - | - | 0.1 or less | - | - |

| | | Example 1 | Example 2 | Example 3 | Example 4 | Example 5 | Example 6 | Example 7 | Example 8 | Comparative Example 1 | Comparative Example 2 | Comparative Example 3 |
|---|---|---|---|---|---|---|---|---|---|---|---|---|
| Multilayer film | Carbon dioxide permeability [mL/(m²/day/atm)] | 103,000 | 103,000 | 103,000 | 79,000 | 79,000 | 69,900 | 51,900 | 101,000 | - | - | - |
| | Oxygen permeability [mL/(m²/day/atm)] | 25,800 | 25,800 | 25,800 | 20,600 | 20,600 | 21,200 | 13,900 | 24,200 | - | - | - |
| | Hydrogen permeability [mL/(m²/day/atm)] | - | - | - | - | 109,000 | - | - | - | - | - | - |
| | Moisture permeability [g/m²·day] | - | - | - | - | 57 | - | - | - | - | - | - |

EP 4 302 997 A1

As shown in Table 1, the carbon dioxide permeability was 70,000 mL/m$^2$•day•atm or more, and the oxygen permeability was 20,000 mL/m$^2$•day•atm or more in each of the Examples, using a multilayer film which included a layer including a 4-methyl-1-pentene-based polymer, and a layer including a polyolefin, and in which the layer including a 4-methyl-1-pentene-based polymer and the layer including a polyolefin contacted each other at at least a portion thereof, and in which the inter-layer adhesion strength between the layer including a 4-methyl-1-pentene-based polymer and the layer including a polyolefin waa 0.5 N/15 mm or more, and in which a heat-seal strength if from 3 N/15 mm to 15 N/15 mm was exhibited when two of the layers that included a polyolefin were brought into contact with each other and heat-sealed at 120 °C.

Further, the multilayer film of each of the Examples exhibited a heat-seal strength at 120 °C of 3 N/15 mm or more, which demonstrates that the multilayer film has excellent heat-sealability at low temperatures.

In contrast, heat-sealing could not be achieved in Comparative Example 1, which did not include a polyolefin layer. In Comparative Example 2 and 3, in which the interlayer adhesion strength was lower than 0.5 N/15 mm, the layers did not adhere to each other, and a multilayer film could not be obtained.

The disclosures of Japanese Patent Application No. 2021-032693, filed March 2, 2021, and Japanese Patent Application No. 2021-182811, filed November 9, 2021, are incorporated herein by reference in their entirety.

All publications, patent applications, and technical standards mentioned in this specification are herein incorporated by reference to the same extent as if each individual publication, patent application, or technical standard was specifically and individually indicated to be incorporated by reference.

**Claims**

1. A multilayer film, comprising a layer that includes a 4-methyl-1-pentene-based polymer, and a layer that includes a polyolefin, wherein the layer that includes a 4-methyl-1-pentene-based polymer and the layer that includes a polyolefin contact each other at at least a portion thereof, an inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

2. The multilayer film according to claim 1, wherein the layer that includes a 4-methyl-1-pentene-based polymer has a content of the 4-methyl-1-pentene-based polymer of from 70 parts by mass to 100 parts by mass.

3. The multilayer film according to claim 1 or claim 2, wherein the polyolefin includes at least one selected from the group consisting of an unsaturated carboxylic acid modified ethylene-α-olefin copolymer, an ethylene-unsaturated carboxylic acid-based copolymer, an ethylene-vinyl acetate copolymer, a silane modified ethylene-vinyl acetate copolymer, an ionomer of an unsaturated carboxylic acid modified ethylene-α-olefin copolymer, and an ionomer of an ethylene-unsaturated carboxylic acid-based copolymer.

4. The multilayer film according to any one of claims 1 to 3, wherein the layer that includes a 4-methyl-1-pentene-based polymer has a thickness of from 5 μm to 50 μm.

5. The multilayer film according to any one of claims 1 to 4, wherein the layer that includes a polyolefin has a thickness of from 1 μm to 15 μm.

6. The multilayer film according to any one of claims 1 to 5, wherein at least one side of the layer that includes a 4-methyl-1-pentene-based polymer has been subjected to at least one treatment selected from the group consisting of plasma treatment, corona treatment, ozone treatment, UV treatment, and flame treatment.

7. The multilayer film according to any one of claims 1 to 6, wherein the multilayer film is obtained by extrusion-laminating the layer that includes a polyolefin onto the layer that includes a 4-methyl-1-pentene-based polymer.

8. The multilayer film according to any one of claims 1 to 7, wherein the multilayer film is an oxygen permeable multilayer film or a carbon dioxide permeable multilayer film.

9. The multilayer film according to any one of claims 1 to 8, wherein the multilayer film has an oxygen permeability of from 11,000 mL/m$^2$•day•atm to 100,000 mL/m$^2$•day•atm, and a carbon dioxide permeability of from 25,000 mL/m$^2$•day•atm to 250,000 mL/m$^2$•day•atm.

10. The multilayer film according to any one of claims 1 to 9, wherein the multilayer film has a hydrogen permeability of from 10,000 mL/m$^2$•day•atm to 300,000 mL/m$^2$•day•atm.

11. The multilayer film according to any one of claims 1 to 10, wherein the multilayer film has a moisture permeability of from 10 g/m$^2$•day to 80 g/m$^2$•day.

12. The multilayer film according to any one of claims 1 to 11, wherein irregularities are formed on a surface of the layer that includes a polyolefin.

13. A container, comprising the multilayer film according to any one of claims 1 to 12, wherein the container is configured to be sealed by heat-sealing the layers that include a polyolefin included in the multilayer film(s).

14. A pack for cell culture, comprising the multilayer film according to any one of claims 1 to 12.

15. A method of producing a multilayer film, the method comprising:

a treatment step of performing a surface treatment on at least one side of a base material that includes a 4-methyl-1-pentene-based polymer; and
a lamination step of laminating a polyolefin onto the side of the base material that has been subjected to the surface treatment, to produce a multilayer film,
wherein, in the multilayer film, an inter-layer adhesion strength between the layer that includes a 4-methyl-1-pentene-based polymer and the layer that includes a polyolefin is 0.5 N/15 mm or more, and a heat-seal strength of from 3 N/15 mm to 15 N/15 mm is exhibited in a case in which two of the layers that include a polyolefin are brought into contact with each other and heat-sealed at 120 °C.

16. The method of producing a multilayer film according to claim 15, wherein the surface treatment includes at least one treatment selected from the group consisting of plasma treatment, corona treatment, ozone treatment, UV treatment, and flame treatment.

17. The method of producing a multilayer film according to claim 15 or claim 16, wherein the lamination step includes subjecting the polyolefin to ozone treatment, and laminating the polyolefin onto the side of the base material that has been subjected to the surface treatment while a side of the polyolefin that has been subjected to the ozone treatment contacts the side of the base material that has been subjected to the surface treatment.

18. The method of producing a multilayer film according to any one of claims 15 to 17, wherein the lamination step is a step of laminating the polyolefin onto the base material that includes a 4-methyl-1-pentene-based polymer, using extrusion lamination.

## INTERNATIONAL SEARCH REPORT

| International application No. |
| --- |
| **PCT/JP2022/008633** |

| A. | CLASSIFICATION OF SUBJECT MATTER |
| --- | --- |

***B32B 27/32***(2006.01)i
FI: B32B27/32 E

According to International Patent Classification (IPC) or to both national classification and IPC

| B. | FIELDS SEARCHED |
| --- | --- |

Minimum documentation searched (classification system followed by classification symbols)

B32B27/32

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 9-268243 A (MITSUI PETROCHEM. IND., LTD.) 14 October 1997 (1997-10-14) claims, paragraphs [0034]-[0036], [0040], [0048], [0055]-[0058], [0060], [0065], [0081], examples | 1, 3-5, 7-11, 13, 15, 18 |
| Y | claims, paragraphs [0034]-[0036], [0040], [0048], [0055]-[0058], [0060], [0065], [0081], examples | 6, 12, 14, 16, 17 |
| A | entire text | 2 |
| Y | JP 2020-15279 A (DAINIPPON PRINTING CO., LTD.) 30 January 2020 (2020-01-30) paragraphs [0020], [0038] | 6, 12, 14, 16, 17 |
| Y | JP 2018-62073 A (TOPPAN PRINTING CO., LTD.) 19 April 2018 (2018-04-19) paragraph [0037] | 12, 14 |
| Y | JP 2018-75822 A (MITSUI CHEMICALS, INC.) 17 May 2018 (2018-05-17) paragraph [0099] | 14 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 April 2022** | **26 April 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2022/008633** |

**C.     DOCUMENTS CONSIDERED TO BE RELEVANT**

| Category* | Citation of document, with indication, where appropriate, of the relevant passages | Relevant to claim No. |
| --- | --- | --- |
| X | JP 61-29540 A (MITSUI CHEMICALS, INC.) 10 February 1986 (1986-02-10) claims, page 4, lower left column, the last paragraph to lower right column, first paragraph, examples | 1, 3-5, 7, 15, 18 |
| Y | claims, page 4, lower left column, the last paragraph to lower right column, first paragraph, examples | 6, 12, 16, 17 |

Form PCT/ISA/210 (second sheet) (January 2015)

## INTERNATIONAL SEARCH REPORT
### Information on patent family members

International application No.

**PCT/JP2022/008633**

| Patent document cited in search report | | | Publication date (day/month/year) | Patent family member(s) | Publication date (day/month/year) |
|---|---|---|---|---|---|
| JP | 9-268243 | A | 14 October 1997 | US 5973077 A claims, column 5, paragraphs [0002]-[0005], [0009], column 6, eighth paragraph, column 7, paragraphs [0003]-[0007], [0009], examples | |
| JP | 2020-15279 | A | 30 January 2020 | (Family: none) | |
| JP | 2018-62073 | A | 19 April 2018 | (Family: none) | |
| JP | 2018-75822 | A | 17 May 2018 | (Family: none) | |
| JP | 61-29540 | A | 10 February 1986 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11301691 A
- JP 2000189051 A
- JP 2015224037 A
- WO 0153369 A
- WO 0127124 A
- JP H3193796 A

- JP H0241303 A
- WO 14050817 A
- WO 2014042249 A
- WO 2006057361 A
- JP 2021032693 A
- JP 2021182811 A